# FASCICULE DE BREVET EUROPEEN

(11) **EP 0 313 487 B1**
(45) Date de publication et mention de la délivrance du brevet: **17.02.1993**
(21) Numéro de dépôt: 88420361.3
(22) Date de dépôt: 21.10.1988
(51) Int. Cl.: A61F 13/06

(54) **Dispositif adapté pour le traitement des entorses, en particulier des chevilles, ainsi que pour la prévention de certaines de leurs complications**
Vorrichtung zur Behandlung von Verstauchungen, insbesondere der Sprunggelenke sowie zur Vermeidung von dabei auftretenden bestimmten Komplikationen
Device adapted for the treatment of sprains especially ankle sprains as well as the prevention of some of their complications

(30) Priorité: 21.10.1987 FR 8715019; 22.04.1988 FR 8805881
(43) Date de publication de la demande: 26.04.1989
(73) Titulaire: Bertheas, Michel, F-42170 St Just St Rambert (FR)
(72) Inventeur: Bertheas, Michel, F-42170 St Just St Rambert (FR)
(74) Mandataire: Dupuis, François

(56) Documents cités:
- DE-A- 1 015 364
- DE-C- 196 847
- FR-A- 1 111 157
- US-A- 1 477 357
- US-A- 3 680 549
- US-A- 4 590 932

## Description

L'invention a pour objet un dispositif adapté pour le traitement des entorses, en particulier des chevilles ainsi que pour la prévention de certaines de leurs complications.

On rappelle que ces entorses se caractérisent sur le plan anatomique par une atteinte des ligaments et de la capsule articulaire, soit élongation, soit rupture partielle ou totale.

Ces lésions peuvent s'accompagner d'un épanchement sanguin péri-articulaire et intra-articulaire qui produit d'importants troubles vaso-moteurs locaux.

Cliniquement, elles sont caractérisées par une vive douleur, un gonflement local rapide et une impotence fonctionnelle proportionnelle à la lésion capsulo-ligamentaire. La palpation trouve un point douloureux précis au niveau des formations ligamentaires lésées. Secondairement peut apparaître une échymose témoin de l'épanchement sanguin. Dans les entorses graves peuvent s'y ajouter des laxités anormales témoins mécaniques de la rupture capsulaire et ligamentaire. Ces laxités exagérées anormalement peuvent être à l'origine d'arthrose.

De manière plus détaillée, suite à une entorse une hémorragie s'établit par déchirure capsulo-ligamentaire et des artérioles nourricières. L'hémorragie a peu de place pour s'étaler, de sorte que l'hématome prend la forme restreinte d'une "coquille d'oeuf". L'hématome décolle les plans des tissus et a tendance à se propager sur toute la région proximale à la lésion et principalement au niveau du pied et de sa plante. Cette effraction dans les tissus voisins de l'hématome provoque des désordres dans la circulation de retour veineuse et lymphatique ; cela crée des perturbations dans les pressions capillaires locales et veineuses en modifiant tous les gradients de pression. Cette perturbation des pressions physiologiques peut entraîner un oedème.

Pour tenter de remédier aux conséquences de l'hématome localisé, on peut évacuer ce dernier par ponctionnement afin de diminuer la pression mécanique surajoutée. Cette méthode est en fait peu utilisée.

Pour diminuer l'hémorragie, on utilise plus généralement un bandage compressif qui est disposé le plus serré possible sur l'hématome. On ajoute systématiquement un glaçage de la région lésée à titre antalgique et anti-hémorragique. Cette méthode est efficace mais dangereuse car en comprimant le pied et la cheville de façon importante et prolongée, on risque de créer un phénomène de garrot circulatoire diminuant considérablement le flux sanguin. Pour éviter ce risque, le bandage est généralement laissé en place pendant une période de trois quarts d'heure environ sous surveillance puis enlevé pour être remplacé par un bandage moins compressif laissé au moins 24 heures. Cette méthode présente plusieurs inconvénients. Elle exige en particulier une surveillance constante de la part du soigneur. Elle présente une difficulté dans la régulation dans la force de pression exercée sur l'hématome par le bandage compressif.

Outre les bandages traditionnels, on utilise des pommades ayant un effet essentiellement sur la douleur, la résorption de l'hématome se faisant dans le temps.

On connaît, au titre de l'art antérieur, le Brevet US 4.590.932 WILKERSON. Ce document propose un ensemble de chevillère de contrôle d'hématome à la suite d'une entorse à la cheville. Cet ensemble comprend un patin mousse thermoplastique ayant une configuration en forme de U. Ce patin de par sa forme en U entoure la malléole et comprend parses branches orientées vers le haut de la cheville deux bandes définissant un canal. De manière tres particulière ce patin est posé encore chaud sur la cheville du patient et il se conforme à la forme particulère de la cheville et au pied pour assurer une compression sur la surface blessée. En outre, ce patin est associé à une chevillère de forme particulière, et on prévoit un bandage complémentaire pour assurer la contention. Il n'y a pas, dans ces conditions, évacuation de l'hématome. L'ensemble précité a pour but et fonction d'empêcher l'hématome d'apparaître au niveau de la cheville. En outre, selon le Brevet US précité, on peut appliquer un degré sélectionné de contention.

Le but recherché selon l'invention, est donc de trouver un dispositif qui appliqué autour de la cheville, puisse circonscrire l'hématome et accélérer son évacuation en favorisant un drainage sur une surface anatomique la plus large possible dans les tissus sains.

La solution apportée simple, facile à mettre en oeuvre est d'un intérêt thérapeutique confirmé par les essais effectués.

Selon la présente invention, le dispositif adapté pour le traitement des entorses des chevilles comprend :
- un support de contention comprenant une première zone destinée à s'adapter sur la partie jambe de l'utilisateur, et une seconde zone qui viendra sur la malléole lorsque le dispositif est en place,
- un moyen d'irrigation et d'évacuation de l'hématome comprenant au moins deux bandes définissant entre elles au moins un canal et dont la partie inférieure est positionnée au moins de part et d'autre de ladite seconde zone du support et dont la partie supérieure est positionnée sur le support en se prolongeant le long de sa partie jambe,
le dispositif étant conformé pour exercer une force de pression sur l'hématome de manière à assurer l'irrigation et l'évacuation de l'hématome vers la partie supérieure de la jambe en résorbant naturellement ledit hématome à l'endroit de l'entorse de la cheville et en assurant une diffusion de l'hématome sur les surfaces tissulaires les plus larges possibles.

Selon une autre caractéristique, le dispositif est incorporé à un support de contention, élastique ou non tel que bas, chaussette.

Selon l'invention, dans une mise en oeuvre particulière, le dispositif est remarquable en ce que le dispositif à plusieurs bandes formant bourrelets définissant des canaux, est intégré à partir et près de l'une des extrémités d'une bande élastique sur une certaine longueur dénommée partie réceptrice, ladite bande étant de grande longueur et s'enroulant autour du cou de pied et par spires progressives le long de la jambe ; l'extrémité de la bande présente une bordure transversale formant bandeau agencé avec des moyens de fixation assurant son maintien sur la jambe ; l'autre extrémité de la bande présente ou non des moyens complémentaires de fixation et de liaison et se positionne après enroulement eu égard à la bordure transversale.

Selon une autre caractéristique de l'invention, le dispositif est remarquable en ce que la bordure transversale formant bandeau est prolongée dans un plan perpendiculaire à celui de la bande par au moins une bride susceptible d'entourer la jambe et d'autoriser par fixation sur elle-même ou sur le bandeau agencé avec des moyens de fixation sur l'une de ses faces, le positionnement de la bande.

Ces caractéristiques et d'autres encore ressortiront bien de la suite de la description.

Pour fixer l'objet de l'invention illustré d'une manière non limitative aux figures des dessins où :
- la figure 1 est une vue du dispositif selon l'invention, dans une première forme de réalisation appliquée sur un bas, autorisant le contention de l'hématome et son évacuation vers le haut de la jambe,
- la figure 2 est une vue en coupe selon la ligne II-II de la figure 1,
- la figure 3 est une vue du dispositif selon l'invention dans une autre forme de réalisation appliquée sur un bas,
- la figure 4 est une vue en coupe selon la ligne IV-IV de la figure 3,
- la figure 5 est une vue illustrant le support dudit dispositif selon l'invention agencé avec une bande complémentaire de contention,
- la figure 6 est une vue à caractère schématique illustrant en variante le cheminement et l'orientation du dispositif du côté externe de la jambe,
- les figures 7, 8 et 9 sont des vues à caractère schématique illustrant diverses formes du dispositif,
- la figure 10 est un exemple d'application dudit dispositif sur un bas.
- la figure 11 est une vue en perspective du dispositif selon l'invention dans une première mise en oeuvre, au début de sa mise en place sur la jambe,
- la figure 12 est une vue partielle à grande échelle illustrant la face intérieure du dispositif selon la figure 11, appliqué sur une bande support,
- la figure 13 est une vue en coupe transversale selon la ligne 13.13. de la figure 2,
- la figure 14 est une vue en coupe transversale selon la ligne 14.14. de la figure 13 illustrant l'apposition du dispositif et son support,
- la figure 15 est une vue illustrant la fixation du dispositif et de la bande support sur la jambe du l'utilisateur,
- la figure 16 est une vue en variante de positionnement du dispositif et de la bande support sur la jambe,
- la figure 17 est une vue selon la figure 16 illustrant la fixation définitive,
- la figure 18 est une vue en perspective de l'ensemble enroulé et stocké,
- la figure 19 est une vue en variante de réalisation du dispositif appliqué sur une bande support,
- la figure 20 est une vue en coupe partielle selon la ligne 20-20 de la figure 9,
- les figures 21 et 22 sont des vues en variante illustrant de l'extérieur l'apposition du dispositif sur des supports de protection de la jambe du type protège-tibia agencés avec des oreilles recouvrant ou entourant la malléole interne et/ou externe du pied,
- les figures 23 et 24 sont des vues à caractère schématique illustrant un agencement différent de points, parties ou zones en relief formés sur le support pour diriger et écouler l'hématome vers le haut de la jambe.

Afin de rendre plus concret l'objet de l'invention, on le décrit maintenant d'une manière non limitative illustrée aux figures des dessins.

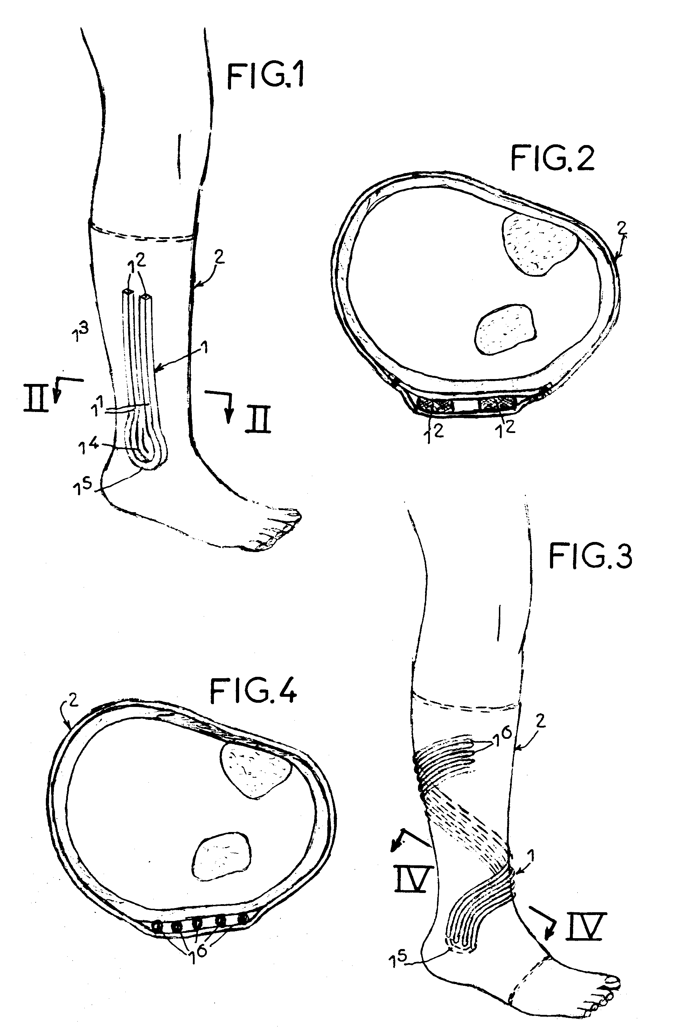

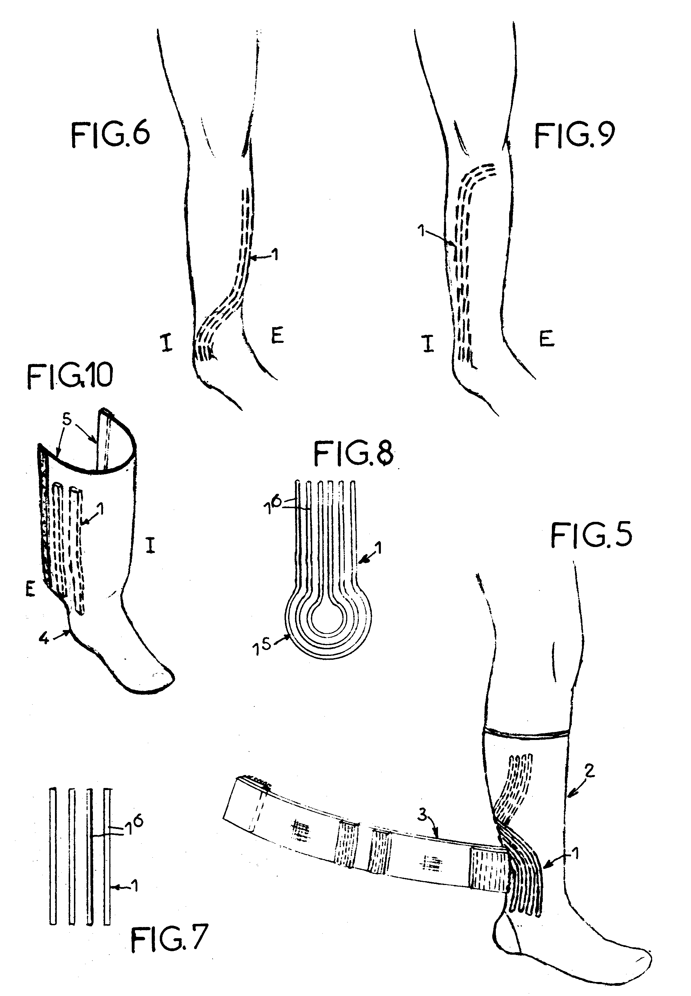

Le concept de l'invention vise à réaliser un dispositif (1) agencé d'au moins deux bandes qui, dans leur partie inférieure (1-1) sont positionnées sur tout ou partie de l'hématome à l'endroit de l'entorse de la cheville et se prolongent par leur partie supérieure (1-2) vers le haut le long de la jambe de l'utilisateur en constituant un ou plusieurs canaux (1-3) d'irrigation et d'évacuation de l'hématome vers ladite partie supérieure de la jambe, en résorbant naturellement ainsi ledit hématome à l'endroit de l'entorse de la cheville. Ledit dispositif peut avantageusement, dans sa partie inférieure, entourer l'hématome comme illustré figures 1 et 8 et constituer une zone de contention (1-4) formant un bourrelet (1-5) profilé en U ou C renversé ou à plusieurs branches (1-6) (figure 8) de manière à orienter l'évacuation de l'hématome vers le haut de la jambe. En variante, ce dispositif peut ne pas présenter dans sa partie inférieure de zone de contention périphérique inférieure autour de l'hématome (figures 5 et 7). A la figure 3, a été illustré en traits interrompus courts, un bourrelet (1-5) de retenue et de contention.

Le dispositif peut être réalisé monobloc ainsi qu'illustré figure 1, c'est-à-dire avec deux bandes espacées de plus ou moins grande longueur définissant entre elles une fente ou canal s'éloignant vers le haut de la jambe, et associées entre elles en partie inférieure par une zone de raccordement.

Le dispositif peut étre réalisé également sous forme d'une pluralité de bandes adjacentes délimitant des canaux entre elles. Les bandes peuvent être indépendantes les unes des autres sur toute leur longeur, ou bien présenter dans leur partie inférieure une zone de liaison et de contention.

Le dispositif (1) est applicable dans la zone périphérique de la malléole, externe ou interne, selon les cas et se prolonge vers le haut de la jambe pour canaliser et évacuer l'hématome vers des zones où l'environnement musculaire tendineux est plus réduit et qui sont moins délicates que le région malléolaire.

Les bandes peuvent être orientées vers le haut de la jambe selon une direction rectiligne (figure 7 par exemple). Elles peuvent également être orientées en hélice.

On a représenté non limitativement aux dessins, quelques trajectoires desdites bandes.

Selon la figure 3, le dispositif est placé côté cheville extérieure, s'oriente sur le cou de pied et contourne la jambe en s'élevant progressivement jusqu'à aboutir après un tour d'hélice dans la partie supérieure externe de la jambe. Selon la figure 6, le dispositif est placé côté cheville extérieure et s'oriente sur le cou du pied puis s'élève du côté intérieur de la jambe.

Des dispositions symétriques sont prévues lorsque le dispositif est placé côté intérieur de la cheville.

En faisant référence à la figure 5, le dispositif est placé côté cheville extérieure, s'oriente derrière la jambe et s'élève en hélice ou de manière rectiligne le long du côté intérieur de la jambe.

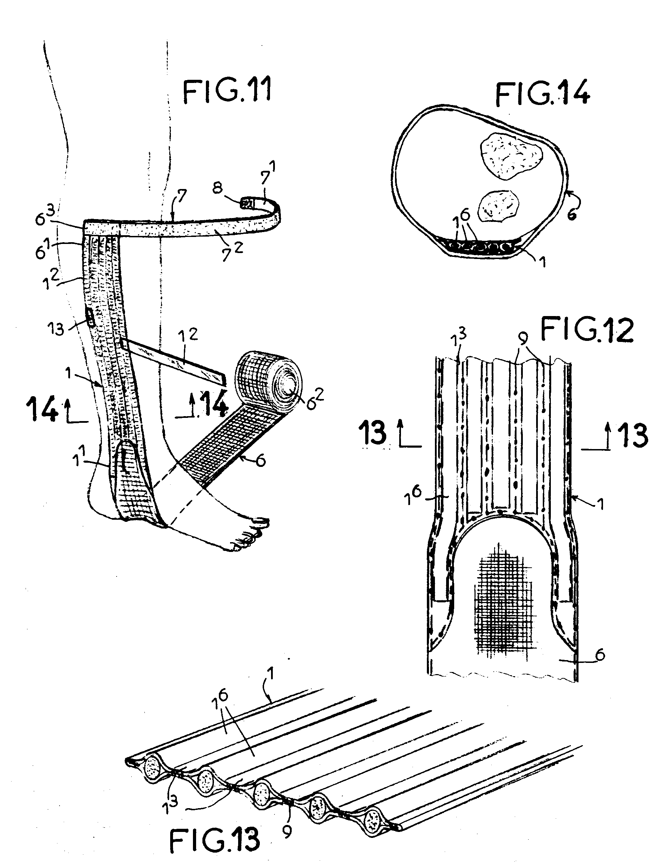

En faisant référence à la figure 9, le dispositif est placé côté cheville extérieure puis se prolonge en remontant le long de la jambe côté extérieur, c'est-à-dire du côté du tibia. L'extrémité supérieure des bandes forme avantageusement mais non limitativement un retour côté intérieur au niveau de l'attache supérieure de l'os du péroné.

Il n'est pas exclu que les bandes puissent s'évaser entre elles vers le haut de la jambe.

On peut envisager également dans une variante de réalisation, un dispositif qui soit réparti simultanément sur les côtés latéraux interne et externe de la jambe.

Le dispositif ainsi décrit peut être réalisé en tous matériaux textiles, plastiques, silicone et autres, avec ou non une plus ou moins grande souplesse de déformation pour éviter l'inconfort. Les bandes peuvent être de toutes sections appropriées. Ce dispositif présente une certaine élasticité lui-même entre les différentes bandes constitutives pour maintenir leur écartement en cours de mouvement de l'utilisateur. Ce dispositif peut présenter lui-même des moyens de liaison et fixation autour de la jambe avec attaches type velcro. Plus généralement, ce dispositif sera incorporé sur un support (2) élastique ou non tel que bas, chaussette, ou équivalent, ouvert ou non à la pointe des orteils, constituant un support de contention. Dans le réalisation de la figure 5, suivant l'effort de contention à exercer, une bande connue en elle-même (3) est rapportée sur le support (2), et peut envelopper selon différentes formes possibles d'enroulement, la partie du pied. Selon la figure 10, le support (2) du dispositif (1) comprend un fourreau (4) ou gaine s'engageant autour du pied, tandis qu'il présente directement rapporté ou en continuité deux ailes (5) susceptibles d'entourer après rapprochement et fermeture, la partie inférieure de la jambe.

L'une des ailes est ainsi agencée avec une pluralité de bandes susceptibles de s'adapter par superposition sur la zone de l'entorse et évacuer l'hématome. En variante, le support peut être ajustable sur toute sa longueur par une fermeture auto-agrippante.

Dans une autre variante illustrée aux figures 11 à 20, le dispositif (1) à plusieurs bandes formant bourrelets (1-6) définissant les canaux (1-3) est intégré sur une certaine longueur, à partir de l'une des extrémités (6-1) d'une bande (6) appelée partie réceptrice (1-7). La bande (6) est de grande longueur et est destinée à venir s'enrouler autour du cou de pied et par spires progressives le long de la jambe. Ladite partie réceptive des bourrelets est d'une longueur correspondant sensiblement à la distance entre la cheville et le genou d'une personne, des tailles pouvant être données par le fabricant. Cette bande est élastique et avantageusement dans les deux sens de sorte qu'elle est parfaitement conformée pour s'adapter à la morphologie du pied et de la jambe de l'utilisateur. l'autre extrémité (6-2) de la bande peut ne présenter aucun moyen particulier et peut être vierge de tous éléments rapportés.

En variante, elle peut être agencée avec des moyens de fixation temporaire du type auto-agrippants ou à pression ou similaires.

Ainsi qu'il apparaît aux figures 11, 12 et 13, l'extrémité (6-1) de la bande présente une bordure transversale (6-3) formant bandeau. Cette bordure présente un côté intérieur lisse (6-4) tandis que son côté extérieur est agencé directement ou de manière rapportée avec des moyens de fixation temporaire tels que moyens auto-agrippants à boucles ou à crochets, moyens de pression ou similaire et dans le cas présent une structure à boucles (6-5). Ledit bandeau (6-3) se prolonge dans un plan perpendiculaire à celui de la bande (6) pour former une sorte de bride (7) supérieure ou laçage. Cette bride est avantageusement agencée avec un côté intérieur (7-1) lisse correspondant au côté (6-4) de la bordure transversale de la bande. Son côté extérieur (7-2) présente sur tout ou partie de sa longueur la partie complémentaire de fixation mâle ou femelle telle que d'une bande auto-agrippante, pression ou similaire en constituant un prolongement homogène de la bordure extérieure (6-5).

Pour assurer la fixation réglable en position de la bride sur elle-même après un enroulement ou sur la partie à boucle du bandeau, il est prévu à l'extrémité de la bride du côté intérieur de celle-ci, une pastille (8) formant moyen de fixation complémentaire aux autres et par exemple un tissu bouclé constituant le moyen complémentaire de fixation.

Cette bride peut présenter éventuellement une certaine élasticité longitudinale. Elle est de grande longueur et permet une adaptation réglable à la morphologie des jambes de chaque utilisateur en permettant un enroulement avec un plus ou moins grand chevauchement formant spire.

La bande élastique (6) reçoit au-delà du bandeau destiné à la fixation de la bride formant ainsi la partie réceptrice de deux ou plusieurs bourrelets longilignes parallèles ou sensiblement, cinq en l'espèce selon les dessins à titre d'exemple, définissant entre eux des canaux d'évacuation de l'hématome. Si les extrémités desdits bourrelets se situent sensiblement dans le même plan côté bandeau (6-3), il a été prévu avantageusement de l'autre côté entourant la cheville, que les deux bourrelets extérieurs soient de plus grande longueur pour entourer morphologiquement ladite cheville. On peut cependant concevoir d'autres aménagements des bourrelets tels que décrits dans la demande de brevet. Les bourrelets sont réalisés en tous matériaux appropriés tels que silicone ou mousse de latex ou matériaux compressibles ou incompressibles similaires.

Les bourrelets sont maintenus par exemple en position par un gainage (8) en tissu, ou autre, les entourant tandis que des lignes de coutures (9) assurent leur séparation les uns des autres tout en définissant les canaux pour l'évacuation de l'hématome.

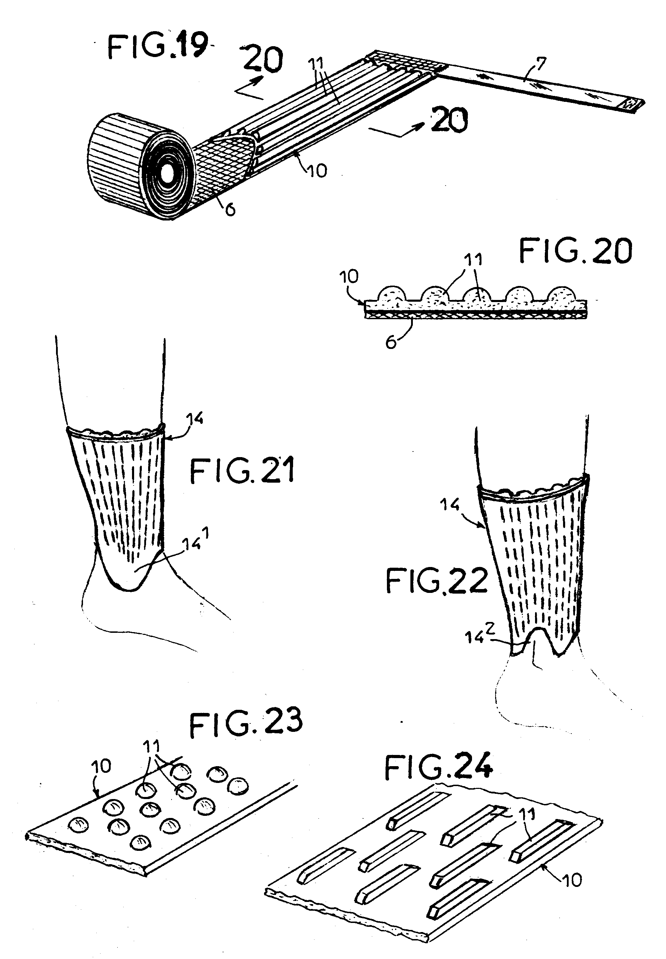

En variante, selon les figures 19 et 20, le dispositif comprend lui-même un support (10) formant orthèse réalisé monobloc en mousse de latex ou matière équivalente et présentant directement formées lors de la fabrication, des parties en saillie (11) destinées à être appliquées contre l'épiderme avec ou sans tissu protecteur complémentaire. Ces parties en saillie ont la forme de bourrelets, tels qu'indiqué précédemment, longilignes, parallèles ou en hélice, ou en variante (figures 23 et 24) selon des parties en relief discontinues, sous forme de pastilles, nervurages et similaires. Ces parties et formes en relief disposées régulièrement ou de manière aléatoire, permettent en combinaison avec l'effort de contention exercé, l'évacuation de l'hématome. On peut prévoir ainsi une pluralité de rangées constituées de parties en relief disposées en quinconce. L'orthèse est collée ou maintenue fixée de toute manière appropriée sur la partie réceptrice de la bande (6).

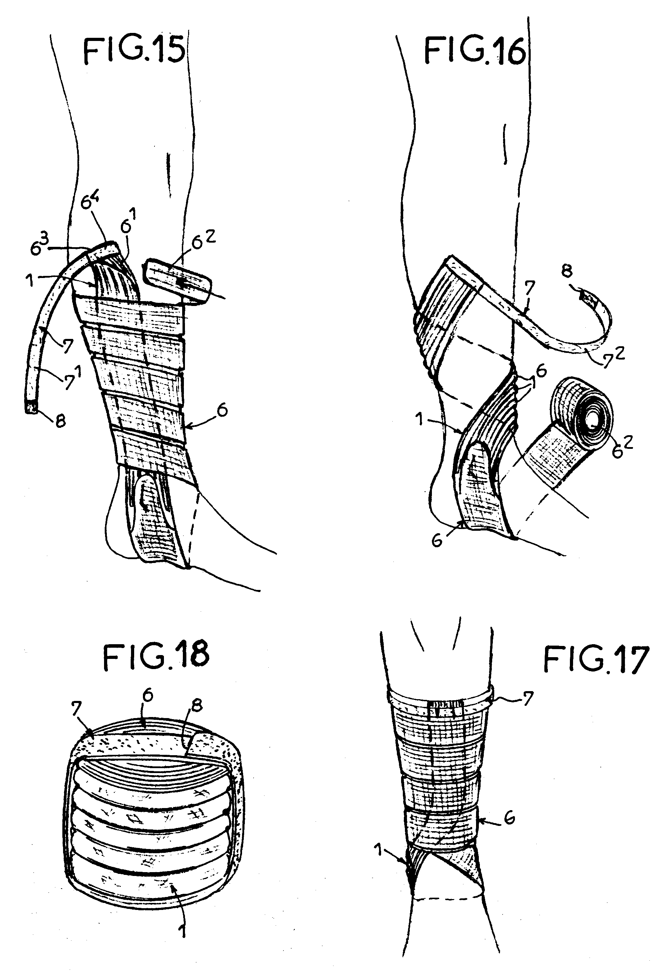

Ainsi réalisé, l'ensemble du dispositif et son support peut très facilement être positionné sur et le long de la jambe. On procède ainsi de la manière suivante. L'utilisateur positionne la partie réceptrice de bande recevant les bourrelets en prenant soin d'entourer dans l'exemple de réalisation la cheville par les bourrelets extrêmes. La bride se trouve à hauteur du genou en étant fixée dans une première position préalable de serrage permettant ainsi de maintenir droite la partie de la bande réceptive le long de la jambe puis d'exercer une tension élastique si nécessaire pour la mise en place correcte de cette partie de bande et donc des bourrelets. L'utilisateur passe ensuite la bande bi-élastique sous le pied (figure 11), puis effectue un ou plusieurs enroulements selon son choix autour du cou de pied en exerçcant une pression de contention avantageusement dégressive de bas en haut, la pression la plus forte étant assurée sur l'hématome. L'utilisateur défile ensuite la bande en formant des spires progressives se chevauchant partiellement en remontant le long de la jambe en recouvrant au fur et à mesure la partie initiale de bande réceptrice des bourrelets, on obtient ainsi un enroulement selon la figure 15 illustrant la bande en fin de phase d'enroulement, une partie de l'extrémité de la bande support de bourrelets n'étant pas recouverte. Une première possibilité consisterait en ce que l'extrémité libre de la bande soit agencée avec des moyens auto-agrippants complémentaires à ceux formés sur la bride pour venir s'accoupler entre eux. Une autre possibilité consiste alors à desserrer et détacher la bride de sorte que l'extrémité libre de la partie bande réceptrice des bourrelets se détende et soit pendante en étant toutefois maintenue en position par la spire supérieure d'enroulement. La phase suivante consiste alors à glisser l'extrémité libre de la bande contre la peau, puis à replacer en position l'extrémité de bande réceptive en maintenant alors en position ladite extrémité libre de bande, puis en replaçant et fixant la bride précitée. L'ensemble du dispositif et son support est donc parfaitement maintenu en place.

Tout en restant dans le cadre de l'invention, on a prévu aux figures 16 et 17 une disposition en variante de la partie réceptrice des bourrelets, celle-ci étant disposée en hélice par rapport à la jambe. L'extrémité inférieure de la partie réceptrice est toujours disposée autour de la cheville, puis passe sur le cou de pied et s'élève progressivement selon une orientation voulue le long de la jambe, la partie libre de la bande vient alors s'enrouler et se fixer de l'une des manières décrites précédemment le long de la bande.

En variante, dans le cadre de l'invention et quelle que soit la mise en forme du dispositif et son positionnement dans un plan sensiblement vertical ou en hélice, on prévoit la possibilité d'adjoindre une seconde bride (12) située sensiblement dans la zone médiane de la partie réceptrice de la bande. Cette seconde bride disposée transversalement constitue un moyen complémentaire de maintien du dispositif sur la jambe du patient. Elle est également agencée avec un ou des moyens de fixation et d'accrochage du type auto-agrippants et similaires, avec un moyen complémentaire (13) disposé le long de la bordure opposée de la partie réceptrice de la bande. Si le dispositif est mis en oeuvre selon le première réalisation des figures, la seconde bride assure une fonction supplémentaire ; lors de sa mise en place, elle permet un étirement sensible en largeur ou une certaine tension de la bande ce qui assure de conserver un certain écartement des bourrelets entre eux lorsque ceux-ci sont en appui sur la face antérieure de la jambe au niveau de l'arête tibiatale en combinaison avec la force de pression des spires de la bande sur lesdits bourrelets.

Sans sortir du cadre de l'invention, l'orthèse monobloc peut être disposée sur des supports de protection des parties de jambes tels que protège-tibia (14) et/ou protège-cheville, agencés avec des oreilles latérales (14-1) ou échancrures (14-2) pour entourer ou contourner les malléoles interne ou externe du pied. l'effort de contention exercé par la fermeture de une ou plusieurs brides associées aux protèges-tibias ou protèges-chevilles ou en disposant une bande bi-élastique entourant par spires successives le support de protection tel que le protège-tibia et/ou cheville, permet un bon maintien de l'ensemble. L'orthèse est agencée comme précédemment avec des bourrelets longilignes ou des points de saillies disposés régulièrement ou irrégulièrement.

Les avantages ressortent bien de l'invention. On souligne en particulier la facilité de réalisation de l'invention, son faible coût de revient, l'adaptation et la mise en place rapide sur la jambe pour l'utilisateur sans aide extérieure. En outre, l'ensemble est facilement ajustable et réglable. La contention est assurée tout le long de la jambe et elle peut être réglable à tous endroits. On souligne enfin le faible encombrement de l'ensemble monté qui se présente sous la forme d'une bobine, la bride de serrage assurant une fonction complémentaire consistant en la fixation de l'ensemble.

On souligne également que le dispositif selon l'invention permet une résorption plus rapide de l'hématome par une diffusion vers les tissus cellulaires en le canalisant vers des régions plus solides biomécaniquement et moins délicates que la région malléolaire. On évite ainsi toutes les complications temporaires dans la région de l'articulation du pied. Le drainage doit être le plus complet possible pour éviter autant que faire se peut, la collection hématique ou lymphatique source de dégats tissulaires provoqués par effraction au moment de l'hémoragie locale traumatique.

Des premières expériences effectuées, il ressort que l'invention présente un grande intérêt et améliore de manière intéressante le traitement des entorses.

## Revendications

1. Dispositif (1) adapté pour le traitement des entorses des chevilles comprenant :
- un support de contention (2, 6, 10, 14) comprenant une première zone destinée à s'adapter sur la partie jambe de l'utilisateur, et une seconde zone qui viendra sur la malléole lorsque le dispositif est en place,
- un moyen d'irrigation et d'évacuation de l'hématome comprenant au moins deux bandes (1.1, 1.2, 1.6) définissant entre elles au moins un canal (1.3) et dont la partie inférieure (1.1) est positionnée au moins de part et d'autre de ladite seconde zone du support et dont la partie supérieure (1.2) est positionnée sur le support en se prolongeant le long de sa partie jambe,
le dispositif (1) étant conformé pour exercer une force de pression sur l'hématome de manière à assurer l'irrigation et l'évacuation de l'hématome vers la partie supérieure de la jambe en résorbant naturellement ledit hématome à l'endroit de l'entorse de la cheville et en assurant une diffusion de l'hématome sur les surfaces tissulaires les plus larges possibles.

2. Dispositif selon la revendication 1, caractérisé en ce qu'il présente dans la partie inférieure une zone de contention (1-4) destinée à entourer l'hématome formant bourrelet (1-5) et relié aux extrémités inférieures desdites bandes délimitant les canaux d'irrigation et d'évacuation de l'hématome.

3. Dispositif selon la revendication 1, caractérisé en ce que les bandes délimitant une ou plusieurs canalisations présentent un évasement progressif vers le haut.

4. Dispositif selon la revendication 1, caractérisé en ce que les bandes présentent un cheminement en hélice prenant naissance dans ladite seconde zone, s'orientant sur le cou de pied et se prolongeant vers le haut selon une trajectoire rectiligne.

5. Dispositif selon la revendication 1, caractérisé en ce que les bandes présentent un cheminement en hélice prenant naissance dans ladite seconde zone, selon une trajectoire continue de l'hélice.

6. Dispositif selon la revendication 1, caractérisé en ce que les bandes présentent un cheminement en hélice prenant naissance dans ladite seconde zone s'orientant derrière la jambe et se prolongeant vers le haut selon une trajectoire rectiligne.

7. Dispositif selon la revendication 1, caractérisé en ce que les bandes présentent un cheminement en hélice prenant naissance dans ladite seconde zone, s'orientant derrière la jambe et se prolongeant vers le haut selon une trajectoire continue de l'hélice.

8. Dispositif selon la revendication 1, caractérisé en ce que les bandes sont réalisées en matériau plus ou moins souple et ont une capacité élastique d'extension ou de déformation.

9. Dispositif selon la revendication 1 et l'une quelconque des revendications 1 à 6, caractérisé en ce que le support de contention, élastique ou non, est un bas, ou une chaussette.

10. Dispositif selon la revendication 1, caractérisé en ce qu'il comprend plusieurs bandes formant bourrelets (1-6) définissant des canaux, ce dispositif étant intégré à partir et près de l'une des extrémités (6-1) d'une bande élastique sur une certaine longueur dénommée partie réceptrice (1-7), ladite bande (6) étant de grande longueur et s'enroulant autour du cou de pied et par spires progressives le long de la jambe ; l'extrémité (6-1) de la bande présentant une bordure transversale (6-3) formant bandeau agencé avec des moyens de fixation assurant son maintien sur la jambe ; l'autre extrémité (6-2) de la bande présentant ou non des moyens complémentaires de fixation et de liaison et se positionnant après enroulement eu égard à la bordure transversale (6-3).

11. Dispositif selon la revendication 10, caractérisé en ce que la bordure transversale (6-3) formant bandeau est prolongée dans un plan perpendiculaire à celui de la bande par au moins une bride (7) susceptible d'entourer la jambe et autoriser par fixation sur elle-même ou sur le bandeau agencé avec des moyens de fixation sur l'une de ses faces, le positionnement de la bande.

12. Dispositif selon la revendication 11, caractérisé en ce que la bride supérieure (7) est agencée avec un côté intérieur (7-1) lisse correspondant au côté intérieur (6-4) de la bordure transversale de la bande ; le côté extérieur de la bride présentant sur tout ou partie de sa longueur des moyens complémentaires de fixation, en prolongement de ceux réalisés sur la partie bandeau; l'extrémité libre intérieure de la bride présentant une pastille (8) ou équivalent avec des moyens de fixation.

13. Dispositif selon l'une quelconque des revendications 10, 11 et 12, caractérisé en ce que les moyens de fixation disposés sur la partie bandeau (6-3), la bride (7) et éventuellement l'extrémité libre (6-2) de la bande, sont du type auto-agrippants.

14. Dispositif selon l'une quelconque des revendications 10, 11, 12 et 13, caractérisé en ce qu'il comprend près de la zone médiane de la partie réceptrice (1-7), une seconde bride de serrage et maintien autour de la jambe, ladite seconde bride présentant des moyens de fixation et d'accrochage sur une partie complémentaire formée et disposée sur la bande.

15. Dispositif selon la revendication 10, caractérisé en ce que la partie réceptrice de la bande reçoit une pluralité de bourrelets disposés en parallèle ou sensiblement, lesdits bourrelets étant maintenus en position par un gainage les entourant et recouvrant la partie réceptrice, tandis que des lignes de coutures (8) ou similaires assurent la séparation des bourrelets en définissant les canaux d'évacuation de l'hématome.

16. Dispositif selon la revendication 10, caractérisé en ce que la partie réceptrice de la bande reçoit directement ou d'une manière rapportée, une orthèse support monobloc profilée et agencée avec des moyens en saillie de guidage et d'évacuation de l'hématome, ces moyens étant réalisés sous forme d'une pluralité de bourrelets continus et longiligne et/ou parties en relief discontinues réparties de manière régulière ou discontinue sur l'orthèse support ; les bourrelets extrêmes se prolongeant ou non plus bas pour entourer la cheville.

17. Dispositif selon les revendications 14 et 16 ensemble, caractérisé en ce que la seconde bride assure une fonction d'écartement préalable et une mise en tension des bourrelets lors du positionnement de ceux-ci sur l'arête tibiatale.

18. Dispositif selon la revendication 11, caractérisé en ce que la bride (7) assure une fonction de fermeture du dispositif appliqué sur la bande après enroulement en vue de son stockage.

19. Dispositif selon la revendication 1, caractérisé en ce qu'il comprend une orthèse support monobloc profilée et agencée avec des moyens en saillie de guidage et d'évacuation de l'hématome, les moyens étnt réalisés sous forme d'une pluralité de bourrelets continus et longilignes et/ou autres parties en relief discontinues disposées de manière régulière ou aléatoire sur l'orthèse support, ladite orthèse est fixée sur un support de protection de parties de jambes tels que protège-tibia et/ou cheville agencés avec des oreilles ou échancrures pour entourer ou contourner les malléoles interne ou externe du pied.

## Claims

1. Device (1) adapted to treat ankle sprains including :
- a compression support (2, 6, 10, 14) including a first area designed to be adapted to the user's leg and a second area which is applied against the malleolus when the device is in place,
- irrigation and evacuation means for the hematoma including at least two strips (1.1, 1.2, 1.6) defining at least one channel (1.3), the bottom part (1.1) of which is positioned at least either side of the said send area of the support and the top part (1.2) is positioned on the support extending along the leg, the device (1) being designed to apply pressure against the hematoma so as to irrigate and evacuate the hematoma to the top part of the leg by naturally resorbing the hematoma at the area of the sprain of the ankle and distributing the hematoma over the largest possible tissue surfaces.

2. Device according to claim 1 wherein the bottom part has a compression area (1-4) designed to surround the hematoma forming a bulge (1-5) and connected to the bottom ends of the said strips delimiting the irrigation and evacuation channels of the hemotoma.

3. Device according to claim 1 wherein the strips delimit one or several channels with gradual flaring towards the top.

4. Device according to claim 1 wherein the strips have a helical path starting in the said second area, directed to the instep and extending upwards according to a rectilinear path.

5. Device according to claim 1 wherein the strips have a helical path starting in the said second zone, according to a continuous helical path.

6. Device according to claim 1 wherein the strips have a helical path starting in the said second area, directed behind the leg and extending upwards according to a rectilinear path.

7. Device according to claim 1 wherein the strips have a helical path starting in the said second area, directed behind the leg and extending upwards according to a continuous helical path.

8. Device according to claim 1 wherein the strips are made of relatively flexible material and have an elastic extension or deformation capacity.

9. Device according to claim 1 and any one of claims 1 to 6 wherein the compression support, elastic or non-elastic, is hosiery or a sock.

10. Device according to claim 1 wherein it includes several strips forming bulges (1-6) defining channels, this device being integrated from and close to one of the ends (6-1) of an elastic strip over a certain length referred to as the receiving part (1-7), the said strip (6) being long and wound around the instep by progressive coils along the leg, the end (6-1) of the strip has a transversal edge (6-3) forming a band provided with fixing means for it to be held against the leg, the other end (6-2) of the strip possibly having complementary fixing and connecting means and being positioned after winding with regard to the transversal edge (6-3).

11. Device according to claim 10 wherein the transversal edge (6-3) forming a band is extended in a plane perpendicular to that of the strip by at least one strap (7) likely to surrond the leg and enable positioning of the strip, by being fixed onto itself or the band provided with fixing means to one of its sides.

12. Device according to claim 11 wherein the top strap (7) is provided with a smooth inner side (7-1) corresponding to the inner side (6-4) of the transversal edge of the strip, the outer side of the strap having over all or part of its length complementary fixing means, extending beyond those made on the band part, the free inner end of the strap having a pad (8) or the equivalent with fixing means.

13. Device according to any one of claims 10, 11 and 12 wherein the fixing means arranged on the band part (6-3), the strap (7) and possibly the free end (6-2) of the strip, are of the self-gripping type.

14. Device according to any one of claims 10, 11, 12 and 13 wherein it includes, close to the median area of the receiving part (1-7), a second clamping strap around the leg, the said second strap having fixing and gripping means to the complementary part formed and arranged on the strip.

15. Device according to claim 10 wherein the receiving part of the strip takes a plurality of bulges arranged in parallel or substantially in parallel, the said bulges being held in position by sheathing, surrounding them and covering the receiving part, whereas seams (8) or the suchlike provide the separation between the bulges defining evacuation channels for the hematoma.

16. Device according to claim 10 wherein the receiving part of the strip takes, either directly or in a built up manner, a one-piece support orthesis, profiled and provided with projecting guiding and evacuation means for the hematoma, these means being designed in the form of a plurality of bulges of the continuous and long type and/or raised, discontinuous parts distributed regularly or discontinuously over the support orthesis, the end bulges either extending or not downwards so as to surround the ankle.

17. Device according to claims 14 and 16 together wherein the second strap provides a prior separating and tensioning function for the bulges when they are positioned against the tibial ridge.

18. Device according to claim 11 wherein the strap (7) provides a closing function of the device applied against the strip after winding with a view to being stored.

19. Device according to claim 1 wherein it includes a one-piece support orthesis, profiled and provided with projecting guiding and evacuation means for the hematoma, the means being designed in the form of a plurality of bulges of the continuous and long type and/or other raised discontinuous parts arranged in a regular or random manner on the support orthesis, the said orthesis is fixed onto a protective support for parts of leg such as shin and/or ankle pad, provided with lugs or notches to surround or contour the internal or external malleolus of the foot.

## Patentansprüche

1. Vorrichtung (1), geeignet für die Behandlung von Fußknöchelverstauchungen, mit :
- einem stützenden Träger (2, 6, 10, 14) mit einem ersten Abschnitt zum Anpassen an die Unterschenkelpartie des Benutzers, und einen zweiten Abschnitt, der im angelegten Zustand der Vorrichtung auf dem Malleolus zu liegen kommt,
- einem Mittel für die Durchblutung und für die Entleerung des Hämatoms mit mindestens zwei Streifen (1.1, 1.2, 1.6), die zwischen sich mindestens einen Kanal (1.3) abgrenzen und deren unterer Bereich (1.1) mindestens beiderseits des genannten zweiten Abschnittes des Trägers positioniert ist, und deren oberer Bereich (1.2) am Träger positioniert ist und sich dabei entlang seiner Unterschenkelpartie erstreckt,
wobei die Vorrichtung (1) so ausgebildet ist, daß sie eine Druckkraft auf das Hämatom derart ausübt, daß die Durchblutung und die Entleerung des Hämatoms in Richtung der oberen Partie des Unterschenkels gewährleistet ist, wobei das genannte Hämatom an dem Punkt der Fußknöchel-Verstauchung natürlich aufgesaugt und eine Verteilung des Hämatoms auf möglichst breitflächige Gewebebereiche sichergestellt wird.

2. Vorrichtung nach Anspruch 1, dadurch gekennzeichnet, daß er im unteren Bereich eine Stützzone (1.4) zum Umgeben des Hämatoms aufweist, die einen Wulst (1.5) bildet und mit den unteren Enden der genannten Streifen verbunden ist, welche die Kanäle für die Durchblutung und für die Entleerung des Hämatoms abgrenzen.

3. Vorrichtung nach Anspruch 1, dadurch gekennzeichnet, daß die eine oder mehrere Kanal-Leitungen abgrenzenden Streifen eine progressive Erweiterung nach oben aufweisen.

4. Vorrichtung nach Anspruch 1, dadurch gekennzeichnet, daß die Streifen einen wendelförmigen Verlauf besitzen, der in dem genannten zweiten Abschnitt beginnt, am Spann entlang verläuft und sich geradlinig nach oben verlängert.

5. Vorrichtung nach Anspruch 1, dadurch gekennzeichnet, daß die Streifen einen wendelförmigen Verlauf besitzen, der in dem genannten zweiten Abschnitt gemäß einer fortlaufenden Linie der Wendel beginnt.

6. Vorrichtung nach Anspruch 1, dadurch gekennzeichnet, daß die Streifen einen wendelförmigen Verlauf besitzen, der in dem genannten zweiten Abschnitt beginnt, hinter dem Unterschenkel entlangläuft und sich geradlinig nach oben verlängert.

7. Vorrichtung nach Anspruch 1, dadurch gekennzeichnet, daß die Streifen einen wendelförmigen Verlauf besitzen, der in dem genannten zweiten Abschnitt beginnt, hinter dem Unterschenkel entlangläuft und sich gemäß einer fortlaufenden Linie der Wendel nach oben verlängert.

8. Vorrichtung nach Anspruch 1, dadurch gekennzeichnet, daß die Streifen aus mehr oder weniger weichem Material hergestellt und elastisch dehn- bzw. verformbar sind.

9. Vorrichtung nach Anspruch 1 sowie einem der Ansprüche 1 bis 6, dadurch gekennzeichnet, daß der elastische oder nichtelastische stützende Träger ein Strumpf oder eine Socke ist.

10. Vorrichtung nach Anspruch 1, dadurch gekennzeichnet, daß sie mehrere Streifen besitzt, die Wulste (1.6) bilden, die Kanäle abgrenzen, wobei diese Vorrichtung ausgehend von und nahe bei einem der Enden (6.1) eines elastischen Bandes über eine bestimmte Länge, den sogenannten Aufnahmebereich, integriert ist, wobei das genannte Band (6) von großer Länge ist und um den Spann und in progressiven Windungen längs des Unterschenkels gewickelt wird; das Ende (6.1) des Bandes ist mit einer Querkante (6.3) versehen, welche eine Binde mit Befestigungsmitteln zum Festhalten am Unterschenkel bildet; das andere Ende (6.2) des Bandes ist mit oder ohne zusätzlichen Mitteln zur Befestigung und Verbindung ausgeführt, die nach dem Umwickeln in Bezug auf die Querkante (6.3) positioniert werden.

11. Vorrichtung nach Anspruch 10, dadurch gekennzeichnet, daß die eine Binde bildende Querkante (6.3) in einer senkrecht zur Bandebene stehenden Ebene durch mindestens einen Gurt (7) verlängert ist, welcher den Unterschenkel umfassen und durch Befestigen der Gurtes auf dem Gurt selbst oder auf der auf einer Seite mit Befestigungsmitteln ausgestatteten Binde das Positionieren des Bandes ermöglichen kann.

12. Vorrichtung nach Anspruch 11, dadurch gekennzeichnet, daß der obere Gurt (7) mit einer glatten Innenseite (7.1) versehen ist, welche der Innenseite (6.4) der Querkante des Bandes entspricht; die Außenseite des Gurtes weist über seine gesamte oder einen Teil seiner Länge ergänzende Befestigungsmittel in der Verlängerung der an der Binde gefertigten Befestigungsmittel auf; am freien inneren Ende des Gurtes befindet sich ein Scheibchen (8) oder ein gleichwertiges Organ mit Befestigungsmitteln.

13. Vorrichtung nach einem der Ansprüche 10, 11 oder 12, dadurch gekennzeichnet, daß die am Bindenteil (6.3), am Gurt (7) und gegebenenfalls am freien Ende (6.2) des Bandes angeordneten Befestigungsmittel als Kletthaftelemente ausgeführt sind.

14. Vorrichtung nach einem der Ansprüche 10, 11, 12 oder 13, dadurch gekennzeichnet, daß sie nahe bei der mittleren Zone des Aufnahmebereichs einen zweiten Gurt zum Festziehen und Festhalten um den Unterschenkel aufweist, wobei der genannte zweite Gurt Mittel zum Befestigen und Anhaften an einem ergänzenden, am Band geformten und angeordneten Teil aufweist.

15. Vorrichtung nach Anspruch 10, dadurch gekennzeichnet, daß der Aufnahmebereich des Bandes eine Mehrzahl von parallel oder annähernd parallel angeordneten Wulsten trägt, wobei die genannten Wulste durch eine sie umgebende und den Aufnahmebereich überdeckende Umhüllung in ihrer Lage fixiert sind, während Nahtlinien (8) die Trennung der Wulste unter Abgrenzung von Kanälen zur Hämatomentleerung sicherstellen.

16. Vorrichtung nach Anspruch 10 dadurch gekennzeichnet, daß der Aufnahmebereich des Bandes direkt oder angesetzt einen profilierten, in einem Stück gefertigten orthopädischen Stützapparat trägt, der mit vorstehenden Mitteln zur Führung und zur Entleerung des Hämatoms ausgestattet ist, wobei diese Mittel in Form einer Mehrzahl durchgehender, schmaler Wulste und/oder unterbrochener erhabener Partien ausgeführt sind, die gleichmäßig oder diskontinuierlich auf dem orthopädischen Stützapparat verteilt sind; die äußeren Wulste sind mit oder ohne Verlängerung nach unten zum Umfassen des Fußknöchels ausgeführt.

17. Vorrichtung nach den Ansprüchen 14 und 16 zusammengenommen, dadurch gekennzeichnet, daß der zweite Gurt dazu dient, eine vorherige Spreizung und ein Spannen der Wulste beim Positionieren der Wulste auf der Schienbeinkante zu bewirken.

18. Vorrichtung nach Anspruch 11, dadurch gekennzeichnet, daß der Gurt (7) dazu dient, die auf dem Band angebrachte Vorrichtung nach dem zum Zweck der Aufbewahrung erfolgten Aufwickeln zu verschließen.

19. Vorrichtung nach Anspruch 1, dadurch gekennzeichnet, daß sie einen profilierten, in einem Stück gefertigten orthopädischen Stützapparat trägt, der mit vorstehenden Mitteln zur Führung und zur Entleerung des Hämatoms ausgestattet ist, wobei die Mittel in Form einer Mehrzahl durchgehender, schmaler Wulste und/oder sonstiger unterbrochener erhabener Partien ausgeführt sind, die gleichmäßig oder regellos auf dem orthopädischen Stützapparat verteilt sind, wobei der orthopädische Apparat an einer mit Zipfeln oder Aussparungen zum Umfassen oder Freilassen des inneren oder äußeren Fußknöchels ausgeführten Halterung zum Schutz von Unterschenkelpartien wie beispielsweise einem Schienbein- und/oder Fußknöchelschützer befestigt ist.
